# EUROPEAN PATENT SPECIFICATION

(11) **EP 3 267 808 B1**
(45) Date of publication and mention of the grant of the patent: **24.04.2019**
(21) Application number: 15714942.8
(22) Date of filing: 11.03.2015
(51) Int. Cl.: A23L 33/10, A23L 2/52, A23F 3/16, A23L 11/00, A23L 29/10

(54) **WATER DISPERSIBLE STEROL/STANOL ENRICHED POLYPHENOL RICH HERBAL TEAS IN AQUEOUS OR POWDERED FORMS TO REDUCE TOTAL AND LDL CHOLESTEROL LEVELS**
WASSERDISPERGIERBARE, MIT STEROL/STANOL ANGEREICHERTE KRÄUTERTEES MIT HOHEM POLYPHENOLGEHALT IN WÄSSRIGER FORM ODER PULVERFORM ZUR REDUZIERUNG DES GESAMT- UND DES LDL-CHOLESTERINSPIEGELS
TISANES RICHES EN POLYPHÉNOLS ENRICHIES EN STÉROLS/STANOLS, DISPERSIBLES DANS L'EAU, SOUS FORME AQUEUSE OU PULVÉRULENTE, SERVANT À RÉDUIRE LES NIVEAUX DE CHOLESTÉROL TOTAL ET LDL

(43) Date of publication of application: 17.01.2018
(73) Proprietor: Tubitak, 06100 Ankara (TR)
(72) Inventor: ALASALVAR, Cesarettin, 41470 Kocaeli (TR); KARADAG, Ayse, 34210 Esenler Istanbul (TR); PELVAN, Ebru, 41470 Kocaeli (TR)
(86) International application number: PCT/IB2015/051765
(87) International publication number: WO 2016/142745

(56) References cited:
- EP-A1- 2 070 429
- WO-A1-2006/020131
- WO-A1-2006/074752
- WO-A1-2009/076742

## Description

### Technical Field

The present invention relates to the field of compositions, either in aqueous or powdered forms, containing polyphenol rich herbal tea extracts and hydrophobic plant sterols/stanols, and the methods for the production thereof. Unless otherwise indicated, the term "plant sterol", as used in this specification and claims, is intended to include both a plant sterol and a plant stanol. Specifically, the present invention relates to plant sterols containing tea drinks with excellent quality retention and stability in which plant sterols/stanols remains stable during storage for a long period. The present invention also relates to plant sterols containing instant tea powders which are easily dispersible in water. This invention further relates to the methods for producing such sterol/stanol containing polyphenol rich herbal tea products either in aqueous or powdered forms and their use in foods, beverages, pharmaceuticals and nutraceuticals, among others. Typical herbal teas include green, black, oolong, and white teas produced from *Camellia sinensis.* Other typical herbal teas include jasmine, linden, rooibos, rosehip, anise, ginseng, chamomile, rosemary, sage, mint, melissa, echinacea, guava leaves and their mixtures.

### Background Art

It has been mentioned that dietary habits and changes in lifestyle lead to an extensive intake of cholesterol contained in food increased blood cholesterol, and thus is a factor in the onset of cardiovascular diseases such as hyperlipidemia, arteriosclerosis, arrhtymia, and myocardial infraction, among others, which currently tend to be increasing globally. Great efforts are focused on reducing the risk of coronary heart disease through dietary interventions. Plant sterols are found to interfere with the absorption of cholesterol into the body and lower its levels in serum.

Plant sterols have chemical structures resembling that of cholesterol and are known to inhibit intestinal cholesterol absorption, thereby reducing the serum cholesterol level, as disclosed in U. S. Pat. No. 5,578,334. Plant sterols are only available to human through plant foods such as vegetable oils, nuts, seeds, cereals, legumes, fruits and vegetables. The current daily intake of plant sterols from conventional foods is estimated to be in the range of 160-400 mg among different populations. The daily doses, considered optimal for the purpose of lowering blood cholesterol levels, are 2-3 g of plant sterols.

However, plant sterols have the disadvantage of poor solubility in both water and oil, and therefore are not easily applicable to foods. Under such circumstances, plant sterol esters had been developed (US Patent No 3,751,569 and WO00061694), which shows an excellent solubility in an oil phase. The drawback to this process, is the use of non-food grade catalysts and reagents to carry out esterification. Another drawback, along with others, is that the kinds of foods, into which plant sterol ester can be incorporated, are limited to specific fat based ones such as margarine, edible oils and mayonnaise. This would be quite inconvenient because of the contradiction that the ingestion of such plant sterol leads to intake of large quantity of edible oil. For this reason, to develop water based products in which plant sterols are easily dispersed or dissolved in is an urgent necessity. There have been several attempts to provide such a product with limited success. Known methods include grinding or milling the plant sterols to increase their solubility (US 3,881,005 and 4,195,084), however micronization of sterol particles alone is not sufficient in order to achieve good incorporability in food matrix, as these particles are poorly wettable, aggregate readily and generally float on aqueous surface.

Therefore, micronization of plant sterols are combined with the additional use of emulsifiers. EP 0897671 B1 includes a method in which high melting point lipid, such as a plant sterol, melted at 150 °C is mixed with non-sterol emulsifier at a ratio of emulsifier to lipid less than 1:2 (w:w), and melting this mixture by heating at 150 °C. Molten mixture was dispersed in the boiled water using high shear and temperature of the mixture is maintained at 95 °C. The phytosterols are reported to have particle size less than 15 µm and preferably less than 10 µm in aqueous dispersions.

In WO2003105611, Coca Cola incorporated plant sterol into fruit beverages without any added emulsifier or thickening agents to avoid the increase in viscosity and detracting from its organoleptic impression in case of using stabiliziers and gums or any formation of ring at the surface of juice or sink to the bottom.

Herbal tea extracts rich in polyphenol can be used to develop different oods and beverages, pharmaceutical, and nutraceutical formulations due to their bioactive properties such as antimicrobial, immuno-stimulatory, anti-carcinogenic, and antiinflammatory activities; and also for its protective effect against metabolic syndrome, e.g., a clustering of several cardiometabolic risk factors including hyperglycaemia, dyslipidaemia and elevated blood pressure, where abdominal obesity and insulin resistance represent core parameters of this cluster. However, it is known that when tea extract rich in polyphenols is cooled down after brewing in hot water, the substances comprise polyphenol-protein and/or caffeine complexes (tea cream) starts to precitipate and this is increased in low pH (pH<5) values which beverages are mostly consumed due to sensory contribution of low acid foods to palatability.

There have been many methods to obtain cold water soluble tea concentrates which have good colour and clarity including alkali treatment to increase the solubility of insoluble complexes and followed by centrifugation to remove any remaining cold-insoluble tea solids (U.S. 3,163,539) or the use of enzymes including tannase and cell wall degrading enzymes (U.S. 4,639,375). Employing alkali chemicals to increase the solubility polyphenol-protein-caffeine complexes would degrade the polyphenols which are the main health related bioactive compounds of tea and applying centrifigation at high speed to increase the sedimented layer would also rise to lose these health related compounds in the final product. The use of enzymes would be expensive and might give tea liquors or infusions with very light colours.

Therefore the incorporation of hydrophobic ingredients (such as sterol) into tea extracts require special treatment to ensure that these ingredients will not increase the system instability and final product will not separate due to density differences, and would have the health related bioactive compounds in its formula and without providing unpleasant effects on the texture of the final products. WO2006074752 (A1), relates to porous sachet comprising plant sterol, an emulsifier, and a particulate component such as tea. Sterol ester and emulsifier are heated together around 60 or 80 °C and cooled to room temperature, mixed with tea leaves and put in a sachet, and after that, are immersed in hot water to get final beverage. The inventors claim that presence of porous sachet would reduce the formation of plant sterol ester droplets on the tea-surface which makes a less attractive impression and, therefore, are preferably avoided or reduced.

### Disclosure of Invention

The present invention was made in the light of the above circumstances. The stability of the system including sterol and tea extract would be different from any system containing sterol and fruit juice extract or any aqueous based foods due to the contribution of polyphenol-protein-caffeine complex in tea extract to the precipitation, even though the water solubility of sterol was improved before addition into the final product.

An object of the present invention is to provide foods and drinks containing an aqueous polyphenol rich herbal tea extract fortified with substantially stable hydrophobic plant sterol, and a production method therefor. To achieve at least one of these or other advantages in accordance with the purpose of the invention, as embodied and broadly described, the inventors have found a process for producing a substantially stable dispersion consisting essentially of hydrohobic plant sterol and aqueous polyphenol rich herbal tea extract.

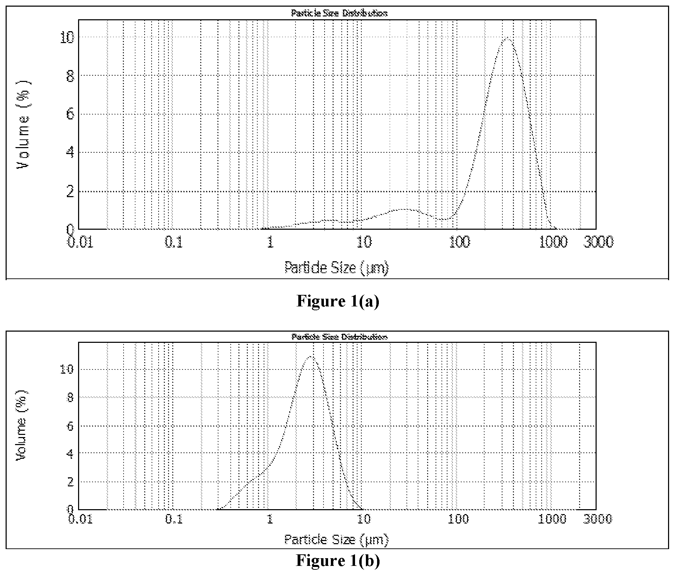

The process involves dispersing or otherwise suspending hydropobic plant sterols into a delivery medium of emulsifier containing an aqueous polyphenol rich herbal tea extract, and exposing the resulting mixture to impact forces by creating high shear. Shear can be generated in preparation of the dispersion of the invention by using a high shear mixer, a colloid mill, a ball mill, high pressure valve homogenizers or a microfluidizer or other mechanical or sonic devices to produce microparticles below 30 µm preferably below 10 µm (**Figure 1**) and which will substantially follow a bell curve distribution. When the particle size of mixture is decreased, the efficacy and stability of delivery medium is enhanced. The higher pressure applied not only reduced the particle size, but also would reduce the size of complexes produced by tea insoluble compounds. The smaller the particle size the better the dispersion, and less sandy feeling in the mouth is obtained.

If desired, for example if the final mixture is used to prepare beverage formulations, additional compounds such as buffers or agents for pH adjustments (e.g. citric acid, tartaric acid, phosphoric acid, acetic acid, salts of any of these acids, sodium hydroxide and sodium bicarbonate), colours, flavours, anti-oxidants (e.g. ascorbic acid, sodium bisulfite, sodium sulfite, butylated hydroxyanisole, butylated hydroxytoluene, alpha-tocopherol) can be included in the dispersion either before or after the homogenization step, for example acidulants may be added before homogenization where as flavours and coloring agents might be added after this step.

In another embodiment, the aqueous medium comprises aqueous herbal tea extracts in combination with emulsifiers and or stabilizers. Emulsifiers/stabilizers can be any type of ionic and nonionic food grade emulsifiers or mixtures thereof, the Hydrophilicity-Lipophilicity Balance (HLB) of which is preferably high (more hydrophilic) about 15 to 18, and which may be selected from sorbitan fatty acid esters, polyoxyethylene sorbitan fatty acid esters, lecithin, sucrose fatty acid esters, polyglycerol fatty acid esters, mono- and diglycerides of fatty acids and their acidic ester or any natural extract with emulsifying properties such as quillaia extract and polysaccharides such as gum arabic, gums, celluose, alginate. The emulsifiers will help to stabilize the system not only due to its sterol content but also by providing higher solubility of polyphenols and reducing the amount of insoluble tea solids, and stability of the tea complex compounds can also be attained by the use of those stabilizers.

In its broader aspect, the solid content of aqueous medium such as solid content of herbal tea extract is from about 100 grams per liter of aqueous medium to 200 grams per liter of aqueous medium and it might be set depending on the aimed concentration in the final product formulation upon dilution.

In yet another embodiment, the aqueous medium where hydropobic plant sterols are suspended into it at varying concentrations, in an amount from about 10 gram to about 200 grams per liter of aqueous medium, is exposed to high shear created by either with an high shear mixer, colloidal mill or high pressure valve homogenizers or microfluidizer to produce microparticles.

In one embodiment, the dispersion before homogenizing may not require heating, however heating is desirable for pasteurization to prevent microbial spoilage. Thus the process may need one or more heating steps and this step may be applied before and/or after homogenization.

In one embodiment, the dispersion before homogenization is optionally heated to temperature of from 20 °C to about 65 or 80 °C for a period of time of from about 0.1 minute to 60 minutes. In another embodiment, the dispersion is optionally heated to 100 °C for a period of time of from 1 to about 20 seconds before homogenization. In yet another embodiment, heated dispersion is cooled to about 25 °C.

In further embodiment, homogenization of the dispersion is conducted in a homogenizer such as a using microfluidizer or high pressure homogenizer. The dispersion may be treated with high shear mixer previously. The homogenization may be operated preferably between 27.579 and 137.895 kPa (4.000 to 20.000 psi). The invention also relates to conducting homogenization at different pressures in single or multiple stages, increasing the number of passes through the chamber to further decrease particle size.

In another embodiment, the aqueous polyphenol rich herbal tea extract may be produced by solvent (generally hot water) extraction of tea leaves with various equipment such as fixed bed reactors, continuous reactors with co-current or counter-current flow or just brewing tea.

By using hot water (about 80-85 °C) and continuous reactors with counter -current flow, more concentrated liquid extract may be produced than in normally brewed tea. The tea extracts produced by continuous reactors with counter -current flow and just brewing tea have tea solids concentration in the region of about 9 to 25 °Brix. The solvent could be acidic buffer which would facilitate separation of insoluble parts and preservation of polyphenols. Insoluble parts in the extract may be separated by centrifugation. The solids concentration or pH of clear centrifugate or filtrate may be adjusted after or before separation process.

In a further embodiment, this aqeuous homogenized dispersion comprising mainly plant sterol and herbal tea extract is either spray dried, or freeze dried, or vacuum dried to get water dispersible sterol fortified powder tea in the presence of excipients such as, but not limited to polysaccharides, maltodextrin, gum arabic, starch and starch derivatives, modified starches, agar, alginic acid, gums, cellulose, microcrystalline cellulose or proteins such as skimmed milk powder, whey proteins. The content of excipients in the dried product is in the range of 2 to 40%, preferably about 5 to 20% by weight. The powder may contain from about 10% to about 60% of plant sterols, preferably about 30% to 40% by weight. The rest of the composition may be composed of the dry matter of aqueous herbal tea extracts and excipients, stabilizers. The fluidity of the powder can be improved through the use of anti-caking agents, such as tricalcium phosphate, talc, stearic acid and its salts, polyethylene glycol or fumed silicon dioxide.

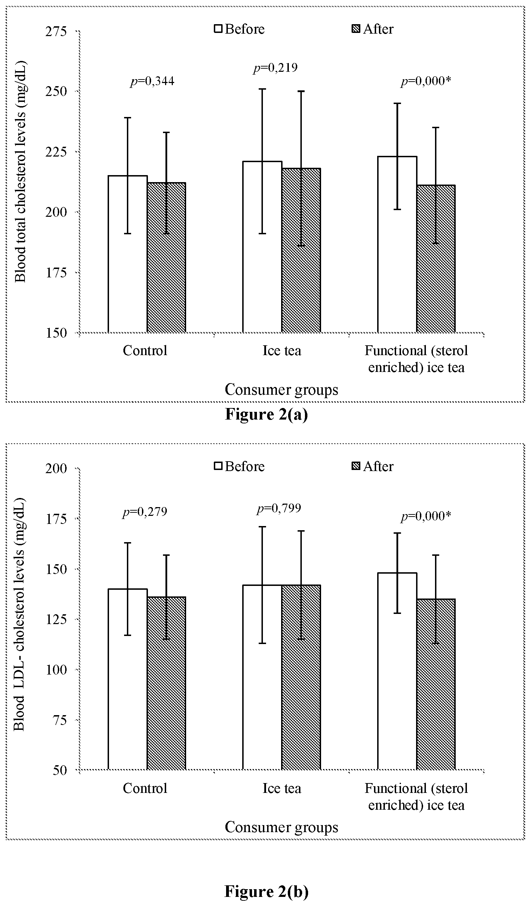

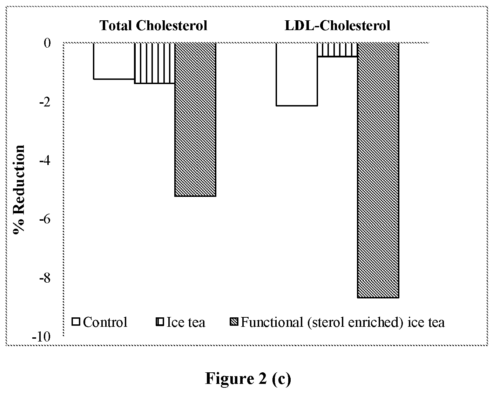

In a further aspect of the invention, this plant sterol enriched herbal tea extract and its dried form can be used in the preparation of pharmaceutical or nutritional products and beverages and the medical applications of such products. The food formulations according to the present invention comprise further constituents, such as tea polyphenols which act independently, simultaneously and/or in synergy with the plant sterols, thus increasing the cholesterol lowering capacity of the final products prepared according to this invention. The consumption of plant sterol enriched herbal tea extract (e.g. black tea) and its dried form in for example beverage formulation would reduce blood total and LD and reduce the oxidative stress of subjects, who were consuming sterol enriched tea bevarage regularly, due to possibly its antioxidative polyphenol content. LDL-cholesterol levels are significantly reduced (**Figure 2a-****c**) in comparison to the consumption of same beverage without sterol or without sterol and tea extract in its composition. Total antioxidant status and oxidative stress of subjects would be improved upon the consumption of sterol enriched black tea product (Table 1)

**Table 1. Change of total antioxidant status, total oxidant status and oxidative stress index of consumers⁽ⁱ⁾ after 4 weeks consumption period of ice tea products**

| | **Control** | | | **Ice tea** | | | **Functional (sterol enriched) ice tea** | | |
|---|---|---|---|---|---|---|---|---|---|
| | **(n=33)** | | | **(n=34)** | | | **(n=32)** | | |
| | ***Before*** | ***After*** | ***p*** | ***Before*** | ***After*** | ***p*** | ***Before*** | ***After*** | ***p*** |
| TAS (mmol/L) | 1.02 ± 0.10 | 0.77 ± 0.34 | 0.000* | 0.92 ± 0.18 | 1.02 ± 0.24 | 0.014* | 0.70 ± 0.13 | 0.75 ± 0.11 | 0.009* |
| TOS (µmol H₂O₂Eq/L) | 9.7 ± 3.8 | 9.6 ± 5.3 | 0.929 | 12.8 (7.5-22.1) | 11.8 (9.9-16.3) | 0.224 | 7.7 (5.0-9.0) | 6.1 (2.3-9.6) | 0.090 |
| OSI (TOS/TAS) | 9.37 ± 3.20 | 13.33 ± 7.49 | 0.011* | 20.40 16.21 | 15.73 ± 11.28 | 0.054 | 12.52 ± 8.47 | 8.41 ± 5.30 | 0.037* |

| | | | | | | | | | |
|---|---|---|---|---|---|---|---|---|---|
| TAS : Total antioxidant status TOS : Total oxidant status OSI: Oxidative stress index (TOS/TAS) *statistically significant at a value of p<0.05 ⁽ⁱ⁾Every group consumed 250 ml of product twice in a day. The product consumed by control group neither includes tea or sterol, but other ingredients in ice-tea; the product consumed by ice-tea group does not include sterol, but includes tea and other ingredients in ice-tea; and the product consumed by functional ice-tea group inclues both sterol (lg/250ml), tea and other ingredients in ice-tea formulation | | | | | | | | | |

### Description of Drawings

**Figure 1 (a)** Particle size of unprocessed plant sterol having a total plant sterol content of 99%, and composed of β-sitosterol, campasterol, stigmasterol, brassicasterol, campastanol, β-sitostanol, the mixture having a melting point of about 138 °C to about 170 °C, derived from vegetable oils and tall oils, and supplied by VitaeNaturals, Vitae Caps, Spain
**Figure 1 (b)** Particle size of processed plant sterol and tea extract mixture
**Figure 2 (a)** Total Cholesterol (mg/dL) levels of 3 groups of consumers after 4 weeks consumption period of ice tea products; blue and red bars represent the total cholesterol levels belongs to the before and after consumption period.
**Figure 2 (b)** LDL-Cholesterol (mg/dL) levels of 3 groups of consumers after 4 weeks consumption period of ice tea products; blue and red bars represent the total cholesterol levels belongs to the before and after consumption period.
**Figure 2 (c)** % of reduction in blood lipid parameters of 3 groups of consumers after 4 weeks consumption period of ice tea products

### EXAMPLES

The invention will now be illustrated by, but is not intended to be limited to, the following examples. In these examples, parts and percentages are by weight, unless noted otherwise.

### Preparation of ice tea beverage from black tea extract fortified with plant sterols

### Example 1

Black tea leaves were extracted by continuous extractor with counter-current flow (Niro Atomizer). Hot water (80-85 °C) was used as extraction solvent with tea: water ratio of 1:5 (w:w) and black tea feed spent 15 minutes inside the extractor. After that, tea extract was centrifuged to reduce tea cream and haze to a specified level (with around 14°Bx value, soluble solids content), and centrifuged black tea extract was mixed with citric acid-sodium citrate buffer to make pH around 3.25. Later on, emulsifiers (Polyoxyethylene (20) sorbitan monooleate and Sucrose fatty acid ester (HLB 16); 0.2 and 0.1% w/v) was dissolved in this mixture and hydrophobic plant sterol (20%) was added into, and the mixture heated to 65 to 80 °C, and it was blended using a high shear mixer (Ultra-Turrax), 1000 rpm for about 5 min. After that, it was homogenized by using high pressure microfludizer (Microfluidics) at 82.737 kPa and 34.474 kPa (12.000 psi and 5.000 psi) to get particle size lower than 10 µm.

This base mixture was, then diluted with water, and flavoured with sugar or artifical/natural sweeteners and aroma compounds (e.g., peach, lemon, mango) to make sterol content 4 g per liter of ice tea beverage having a pH of around 3.45 (citric acid-sodium citrate, 1.6-0.6 g per liter) and, where its final serving size was aimed to have 1 g per 250 ml of beverage. Finally, the beverage was pasteurized at 85-100 °C for 30-60 seconds (or other viable combination of time and temperature), cooled and bottled aseptically and followed by cooling to 20°C.

| **Sterol enriched black ice tea formulation-Base** | Percent (%) |
|---|---|
| Centrifuged black tea extract (°Bx value of 14) | 50 |
| Emulsifier | |
| Polyoxyethylene (20) sorbitan monooleate (HLB 15) | 0.1 |
| Sucrose fatty acid ester (HLB 16) | 0.05 |
| Plant sterol | 20 |
| Buffer to make total of 100 | 29.5 |

| **Sterol enriched black ice tea formulation-Finished product** | grams |
|---|---|
| Sugar | 70 |
| Base | 20 |
| Aroma (e.g. peach) | 0.1 |
| Water | 1000 |

### Example 2

Black tea extract in example 1 could be replaced with green tea extract or any other herbal tea extract. The highest plant sterol added to base mixture to be able to work sufficiently with microfluidizer was around 15-25%; dilution ratio would be around 1 to 40-60 (w:w) to get a product 1 g of plant sterol in 1 serving size of beverage (250 ml), which tea extract would be diluted to having around 3-2.25 g tea per liter when the °Brix ratio in the beginning was around 14. The final solid content of beverage can be set to different values according to sensorial acceptance and °Brix value of herbal tea extract, sterol content of base mixture

### Example 3

### Preparation of sterol enriched powdered tea

The base mixture in Example 1, 2 mixed with gum arabic or gum arabic:maltodextrin (1:4, w:w) at 5% in total weight, can be spray dried with an inlet temperature of 140 °C and temperature at the outlet was 90 °C . The final sterol content of powder produced the mixture from Example 1 was around 50%. Tea extract can also be agglomerated by using fluidized bed reactor at following conditions: air flow rate: 105-80 m³/h, air temperature: 85 °C, exhaust air temperature: 45-50 °C, product temperature: 50-55 °C, spraying pressure: 2.5 bar, pump position: 3-8. Final powder can be mixed with sugar or sweeteners as an ice tea beverage that is ready to drink after redispersion in water.

Powdered tea fortified with plant sterol can be used in any product such as, but not limited to, beverage formulations, nutraceutical preparations, ice-cream, and baked goods among others.

## Claims

1. A process for producing foods and drinks containing one or more aqueous polyphenol rich herbal tea extract fortified with one or more hydrophobic plant sterol wherein the process comprises:
dispersing or otherwise suspending hydrophobic plant sterols into a delivery medium of an emulsifier or a mixture of emulsifiers/ stabilizers, preferably with high HLB values, containing an aqueous polyphenol rich herbal tea extract,
and homogenizing the resulting mixture by a high shear mixer, a colloidal mill, a high pressure valve homogenizer or a microfluidizer.

2. A process according to claim 1 wherein herbal teas include green, black, oolong, and white teas produced from Camellia sinensis or other typical herbal teas including jasmine, linden, rooibos, rosehip, anise, ginseng, chamomile, rosemary, sage, mint, melissa, echinacea, guava leaves and their mixtures.

3. A process according to claim 1 wherein the concentration of the aqueous medium where hydropobic plant sterols are suspended, is from 10 gram to 200 grams hydropobic plant sterols per liter of aqueous medium

4. A process according to claim 1 wherein, the aqueous medium comprises aqueous herbal tea extracts in combination with emulsifiers and/or stabilizers

5. A process according to claim 1 wherein the emulsifiers/stabilizers are ionic and nonionic food grade emulsifiers or mixtures thereof, the Hydrophilicity-Lipophilicity Balance (HLB) of which (HLB) is preferably high (more hydrophilic) about 15 to 18, and which are selected from sorbitan fatty acid esters, polyoxyethylene sorbitan fatty acid esters, lecithin, sucrose fatty acid esters, polyglycerol fatty acid esters, mono- and diglycerides of fatty acids and their acidic ester or any natural extract with emulsifying properties such as quillaia extract and polysaccharides such as gum arabic, gums, cellulose or alginate.

6. A process according to claim 1 wherein the shear is generated by using a high shear mixer, a colloid mill, a ball mill, a high pressure valve homogenizer or a microfluidizer or other mechanical or sonic devices to produce microparticles below 30 preferably below 10 µm and which will substantially follow a bell curve distribution.

7. A process according to claim 1 wherein additional compounds such as buffers or agents for pH adjustments (e.g. citric acid, tartaric acid, phosphoric acid, acetic acid, salts of any of these acids, sodium hydroxide and sodium bicarbonate), colours, flavours, anti-oxidants (e.g. ascorbic acid, sodium bisulfite, sodium sulfite, butylated hydroxyanisole, butylated hydroxytoluene, alpha-tocopherol) are included in the dispersion either before or after homogenization step.

8. A process according to claim 1 wherein the process uses one or more heating steps which are applied before and/or after homogenization.

9. A process according to claim 1 wherein the dispersion before homogenization is optionally heated to temperature of from 20 °C to 65°C for a period of time of from minute to 60 minutes.

10. A process according to claim 1 wherein, the dispersion is optionally heated to 100 °C for a period of time of from 1 to 20 seconds before homogenization.

11. A process according to claim 1 wherein heated dispersion is cooled to about 25 °C.

12. A process according to claim 1 wherein homogenization of the dispersion is conducted in a homogenizer such as a microfluidizer or a high pressure homogenizer.

13. A process according to claim 1 wherein the dispersion is treated with a high shear mixer before homogenization.

14. A process according to claim 1 wherein the homogenization is preferably operated at between 27.579 and 137.895 kPa (4.000 to 20.000 psi).

15. A process according to claim 1 wherein homogenization is conducted at different pressures in single or multiple stages.

16. A process according to claim 1 wherein the aqueous polyphenol rich herbal tea extract is produced by solvent (generally hot water) extraction of tea leaves with various equipment such as fixed bed reactors, continuous reactors with co-current or counter-current flow or just by brewing tea.

17. A process according to claim 16 wherein the solvent is an acidic buffer which facilitates preservation of polyphenols and separation of insoluble parts in the extract which may be separated by a centrifugation process, further wherein the solids concentration or the pH of the centrifugate or filtrate is adjusted after or before the separation process.

18. A process according to claim 1 wherein the aqeuous homogenized dispersion compirising mainly plant sterol and herbal tea extract is either spray dried, or freeze dried, or vacuum dried to get water dispersible sterol fortified powder tea in the presence of excipients such as, but not limited to polysaccharides, maltodextrin, gum arabic, starch and starch derivatives, modified starches, agar, alginic acid, gums, cellulose, mycrocystalline cellulose or proteins such as skimmed milk powder or whey proteins.

19. A process according to claim 18 wherein the content of excipients in the dried product is in the range of 2 to 40%, preferably 5 to 20% by weight.

20. A process according to claim 18 wherein the fluidity of the powder is optionally improved through the use of anti-caking agents, such as tricalcium phosphate, talc, stearic acid and its salts, polyethylene glycol or fumed silicon dioxide.

## Patentansprüche

1. Verfahren zur Herstellung von einen oder mehrere wässerige polyphenolreiche Kräutertee-Extrakte enthaltende, mit einem oder mehreren hydrophoben Pflanzensterinen angereicherten Nahrungsmitteln und Getränken, wobei das Verfahren umfasst:
Dispergieren oder anderweitig Suspendieren der hydrophobe Pflanzensterinen in einem Abgabemedium eines Emulgators oder eines Gemisches aus Emulgatoren / Stabilisatoren, vorzugsweise mit hohen HLB-Werten, enthaltend einen wässrigen, polyphenolreichen Kräuterteeextrakt,
und Homogenisieren des resultierenden Gemisches durch einen Hochscherungsmischer, eine Kolloidmühle, einen Hochdruckventilhomogenisator oder einen Mikrofluidisator.

2. Verfahren nach Anspruch 1, wobei die Kräutertees grüne, schwarze, oolong und weiße Tees einschließen, die aus Camellia Sinensis oder anderen typischen Kräutertees hergestellt werden, einschließlich Jasmin, Linden, Rooibos, Hagebutte, Anis, Ginseng, Kamille, Rosmarin, Salbei, Minze, Melisse , Echinacea, Guavenblätter und deren Mischungen.

3. Verfahren nach Anspruch 1, wobei die Konzentration des wässrigen Mediums, in dem hydrophobe Pflanzensterole suspendiert sind, 10 bis 200 g hydrophobe Pflanzensterole pro Liter von wässriges Medium beträgt.

4. Verfahren nach Anspruch 1, wobei das wässrige Medium wässrige Kräuterteeextrakte in Kombination mit Emulgatoren und / oder Stabilisatoren aufweist.

5. Verfahren nach Anspruch 1, wobei die Emulgatoren / Stabilisatoren ionische und nichtionische Emulgatoren von Lebensmittelqualität oder Mischungen davon sind, das Hydrohilizitäts-Lipohizilitäts-Gleichgewicht (HLB) vorzugsweise etwa 15 bis 18 hoch (hydrophiler) ist und die aus Sorbitanfettsäureester, Polyoxyethylensorbitanfettsäureester, Lecithin, Saccharosefettsäureester, Polyglycerinfettsäureester, Mono- und Diglyceride von Fettsäuren und deren saure Ester oder ein beliebiger natürlicher Extrakt mit emulgierenden Eigenschaften wie Quillaia-Extrakt und Polysaccharide wie Gummi Arabicum, Gummi, Cellulose oder Alginat ausgewählt werden.

6. Verfahren nach Anspruch 1, wobei die Scherung unter Verwendung eines Hochschermischers, einer Kolloidmühle, einer Kugelmühle, eines Hochdruckventil-Homogenisators oder eines Mikrofluidisierers oder anderen mechanischen Geräten oder Schalvorrichtungen zur Herstellung von Mikropartikeln unter 30 µm, vorzugsweise unter 10 µm erzeugt wird und die im Wesentlichen einer Glockenkurvenverteilung folgt.

7. Verfahren nach Anspruch 1, wobei zusätzliche Verbindungen wie Puffer oder Mittel zur Einstellung des pH-Werts (z. B. Zitronensäure, Weinsäure, Phosphorsäure, Essigsäure, Salze einer beliebigen dieser Säuren, Natriumhydroxid und Natriumbikarbonat), Farbstoffe, Aromen, Anti-Agens Oxidationsmittel (z. B. Ascorbinsäure, Natriumbisulfit, Natriumsulfit, butyliertes Hydroxyanisol, butyliertes Hydroxytoluol, alpha-Tocopherol) sind entweder vor oder nach dem Homogenisierungsschritt in die Dispersion eingeschlossen.

8. Verfahren nach Anspruch 1, wobei das Verfahren einen oder mehrere Erwärmungsschritte benutzt, die vor und / oder nach dem Homogenisierung angewendet werden.

9. Verfahren nach Anspruch 1, wobei die Dispersion vor der Homogenisierung optional für eine Zeitdauer von 1 Minute bis 60 Minuten auf eine Temperatur von 20 ° C bis 65 ° C erhitzt wird.

10. Verfahren nach Anspruch 1, wobei die Dispersion vor der Homogenisierung optional für eine Zeitspanne von 1 bis 20 Sekunden auf 100 ° C erhitzt wird.

11. Verfahren nach Anspruch 1, wobei die erhitzte Dispersion auf etwa 25 ° C gekühlt wird.

12. Verfahren nach Anspruch 1, wobei die Homogenisierung der Dispersion in einem Homogenisator wie einem Mikrofluidisator oder einem Hochdruckhomogenisator durchgeführt wird.

13. Verfahren nach Anspruch 1, wobei die Dispersion mit einem Hochscherungsmischer vor der Homogenisierung behandelt wird.

14. Verfahren nach Anspruch 1, wobei die Homogenisierung vorzugsweise bei 27,579 bis 137,895 kPa (4.000 bis 20.000 psi) betrieben wird.

15. Verfahren nach Anspruch 1, wobei die Homogenisierung bei verschiedenen Drücken in einer Stufe oder mehreren Stufen durchgeführt wird.

16. Verfahren nach Anspruch 1, wobei der wässerige, polyphenolreiche Kräuterteeextrakt durch Lösungsmittelextraktion (im wesentlichen heißes Wasser) von Teeblättern mit verschiedenen Ausrüstungen wie Festbettreaktoren, kontinuierlichen Reaktoren mit Gleichstrom oder Gegenstromfluß oder einfach durch Tee Brauen hergestellt wird.

17. Verfahren nach Anspruch 16, wobei das Lösungsmittel ein saurer Puffer ist, der die Konservierung von Polyphenolen und die Abtrennung unlöslicher Teile im Extrakt ermöglicht, die durch ein Zentrifugationsverfahren abgetrennt werden können, wobei weiterhin die Feststoffkonzentration oder der pH-Wert des Zentrifugats oder Filtrats nach oder vor dem Trennungsprozess eingestellt werden.

18. Verfahren nach Anspruch 1, wobei die wässrige homogenisierte Dispersion enthaltende hauptsächlich Pflanzensterol und Kräutertee Extrakt, um in Wasser dispergierbaren, mit Sterin angereicherten Pulvertee in Gegenwart von Hilfsstoffen, wie zum Beispiel, aber nicht darauf beschränkt, Polysaccharide, Maltodextrin, Gummi arabicum, Stärke und Stärkederivate, modifizierte Stärken, Agar, Alginsäure, Gummi, Cellulose, mikrokristalline Cellulose oder Proteine wie Magermilchpulver oder Molkeproteine zu erhalten, entweder sprühgetrocknet oder gefriergetrocknet oder vakuumgetrocknet wird.

19. Verfahren nach Anspruch 18, wobei der Gehalt der Hilfsstoffe im getrockneten Produkt im Bereich von 2 bis 40.%, vorzugsweise von 5 bis 20% liegt nach dem Gewicht.

20. Verfahren nach Anspruch 18, wobei die Fließfähigkeit des Pulvers gegebenenfalls durch die Benutzung von Antibackmitteln wie Tricalciumphosphat, Talkum, Stearinsäure und deren Salzen, Polyethylenglykol oder pyrogenem Siliciumdioxid verbessert wird.

## Revendications

1. Le procédé de production d'aliments et de boissons contenant un ou plusieurs extraits aqueux de tisane riches en polyphénole enrichis avec un ou plusieurs stérols végétaux hydrophobes, dans lequel le procédé comprend:
De disperser ou mettre en suspension des stérols végétaux hydrophobes dans un milieu d'un émulsifiant ou d'un mélange d'émulsifiants / stabilisants de préférence à haute valeur HLB contenant un extrait aqueux de tisane riche en polyphénole,
et d'homogénéiser le mélange résultant d'un mélangeur à cisaillement élevé, d'un broyeur colloïdal, d'un homogénéiseur à vanne haute pression ou d'un microfluidiseur.

2. Le procédé selon la revendication 1, dans lequel les tisanes comprennent des thés verts, noirs, oolongs et blancs produits à partir de Camellia sinensis ou d'autres tisanes typiques comprenant du jasmin, du tilleul, du rooibos, de l'églantier, de l'anis, du ginseng, de la camomille, du romarin, de la sauge, de la menthe, et du mélisse , échinacée, feuilles de goyave et leurs mélanges.

3. Le procédé selon la revendication 1, dans lequel la concentration du milieu aqueux où les stérols végétaux hydrophobes sont en suspension et cette concentration est comprise entre 10 grammes et 200 grammes de stérols végétaux hydrophobes par litre de milieu aqueux.

4. Le procédé selon la revendication 1, dans lequel le milieu aqueux comprend des extraits aqueux de tisane en combinaison avec des émulsifiants et/ou des stabilisants.

5. Le procédé selon la revendication 1, dans lequel les émulsifiants / stabilisants sont des émulsifiants de qualité alimentaire ioniques et non ioniques où leurs mélanges et la balance hydrohilicité-lipohicité (HLB) sont de préférence élevée (plus hydrophile) d'environ 15 à 18, et qui est choisie parmi l'acide gras de sorbitan esters, esters d'acides gras de polyoxyéthylène sorbitan, lécithine, esters d'acides gras de saccharose, esters d'acides gras de polyglycérole, mono- et diglycérides d'acides gras et leur ester acide ou tout extrait naturel ayant des propriétés émulsionnantes telles que l'extrait de quillaia et les polysaccharides tels que la gomme arabique, la gomme, la cellulose ou l'alginate.

6. Le procédé selon la revendication 1, dans lequel le cisaillement est généré en utilisant un mélangeur à haut cisaillement, un broyeur à colloïdes, un broyeur à boulets, un homogénéiseur à vanne haute pression ou un microfluidiseur ou d'autres dispositifs mécaniques ou sonores pour produire des microparticules inférieurs à 30 µm, de préférence inférieurs à 10 µm et qui suivra sensiblement une distribution en courbe en cloche.

7. Le procédé selon la revendication 1, dans lequel des composés supplémentaires tels que des tampons ou des agents pour ajuster le pH (par exemple l'acide citrique, l'acide tartrique, l'acide phosphorique, l'acide acétique, les sels de l'un quelconque de ces acides, l'hydroxyde de sodium et le bicarbonate de sodium), les colorants, les arômes, les oxydants (par exemple l'acide ascorbique, le bisulfite de sodium, le sulfite de sodium, l'hydroxyanisole butyle, l'hydroxytoluène butyle, l'alpha-tocophérol) sont inclus dans la dispersion avant ou après l'étape d'homogénéisation.

8. Le procédé selon la revendication 1, dans lequel le procédé utilise une ou plusieurs étapes de chauffage qui sont appliquées avant et/ou après l'homogénéisation.

9. Le procédé selon la revendication 1, dans lequel la dispersion avant l'homogénéisation est éventuellement chauffée à une température de 20°C à 65°C pendant une durée de 1 à 60 minutes.

10. Le procédé selon la revendication 1, dans lequel la dispersion est éventuellement chauffée à 100°C pendant une période de temps de 1 à 20 secondes avant l'homogénéisation.

11. Le procédé selon la revendication 1, dans lequel la dispersion chauffée est refroidie à environ 25 ° C.

12. Le procédé selon la revendication 1, dans lequel l'homogénéisation de la dispersion est réalisée dans un homogénéisateur tel qu'un microfluidiseur ou un homogénéisateur à haute pression.

13. Le procédé selon la revendication 1, dans lequel la dispersion est traitée avec un mélangeur à cisaillement élevé avant une homogénéisation.

14. Procédé selon la revendication 1, dans lequel l'homogénéisation est effectuée de préférence entre 27,579 et 137,895 pKa (4 000 à 20 000 psi).

15. Le procédé selon la revendication 1, dans lequel l'homogénéisation est réalisée à différentes pressions en une ou plusieurs étapes.

16. Le procédé selon la revendication 1, dans lequel l'extrait aqueux à base de thé riche en polyphénole est produit par extraction au solvant (généralement à l'eau chaude) de feuilles de thé avec divers équipements tels que des réacteurs à lit fixe, des réacteurs continus à flux à co-courant ou à contre-courant juste en infusant du thé.

17. Le procédé selon la revendication 16, dans lequel le solvant est un tampon acide qui facilite la conservation des polyphénoles et la séparation des parties insolubles dans l'extrait qui peut être séparé par centrifugation, dans lequel la concentration en solides ou le pH du centrifuge ou du filtrat est ajusté après ou avant le processus de séparation.

18. Le procédé selon la revendication 1, dans lequel la dispersion aqueuse homogénéisée comprenant principalement du stérol végétal et de l'extrait de tisane est soit séchée par atomisation, soit lyophilisée, soit séchée sous vide pour obtenir du thé en poudre enrichi au stérol dispersable dans l'eau en présence d'excipients tels que les polysaccharides, la maltodextrine, la gomme arabique, les dérivés d'amidon et l'amidon, les amidons modifiés, l'agar-agar, l'acide alginique, les gommes, la cellulose, la cellulose micro-cristalline ou des protéines telles que le lait écrémé en poudre ou les protéines de lactosérum.

19. Le procédé selon la revendication 18, dans lequel la teneur en excipients dans le produit séché est comprise dans l'intervalle de 2 à 40% et de préférence de 5 à 20% en poids.

20. Le procédé selon la revendication 18, dans lequel la fluidité de la poudre est éventuellement améliorée par l'utilisation d'agents anti-agglomérants, tels que le phosphate tricalcique, le talc, l'acide stéarique et ses sels, le polyéthylèneglycole ou le dioxyde de silicium sublimé.
